# EUROPÄISCHE PATENTSCHRIFT

(11) **EP 1 185 709 B1**
(45) Veröffentlichungstag und Bekanntmachung des Hinweises auf die Patenterteilung: **12.05.2004**
(21) Anmeldenummer: 99939320.0
(22) Anmeldetag: 15.06.1999
(51) Int. Cl.: C12Q 1/68, B01J 19/00

(54) **VORRICHTUNG UND VERFAHREN ZUM SPEICHERN VON INFORMATIONEN**
DEVICE AND METHOD FOR STORING INFORMATION
DISPOSITIF ET PROCEDE POUR MEMORISER DES INFORMATIONS

(43) Veröffentlichungstag der Anmeldung: 13.03.2002
(73) Patentinhaber: November Aktiengesellschaft Gesellschaft für Molekulare Medizin, 91056 Erlangen (DE)
(72) Erfinder: BERTLING, Wolf, D-91056 Erlangen (DE)
(74) Vertreter: Gassner, Wolfgang, Dr.
(86) Internationale Anmeldenummer: PCT/DE1999/001738
(87) Internationale Veröffentlichungsnummer: WO 2000/077248

(56) Entgegenhaltungen:
- EP-A- 0 745 690
- WO-A-98/51819
- DE-A- 19 808 884
- DE-A- 19 811 730
- US-A- 5 024 227
- US-A- 5 607 834
- US-A- 5 723 591
- US-A- 5 866 336
- OTA N ET AL.: "Determinaton of interactions between structured nucleic acids by fluorescence resonance energy transfer (FRET): selection of target sites for functional nucleic acids" NUCLEIC ACIDS RESEARCH, Bd. 26, Nr. 3, 1998, Seiten 735-743, XP002131282
- COOPER ET AL: "Analysis of fluorescent energy transfer in duplex and branched DNA molecules" BIOCHEMISTRY,US,AMERICAN CHEMICAL SOCIETY. EASTON, PA, Bd. 29, 1. Januar 1990 (1990-01-01), Seiten 9261-9268, XP002082476 ISSN: 0006-2960
- TYAGI S ET AL: "MOLECULAR BEACONS: PROBES THAT FLUORESCE UPON HYBRIDIZATION" NATURE BIOTECHNOLOGY,US,NATURE PUBLISHING, Bd. 14, 1. März 1996 (1996-03-01), Seiten 303-308, XP000196024 ISSN: 1087-0156

## Beschreibung

Die Erfindung betrifft eine Vorrichtung und ein Verfahren zum Speichern von Informationen, insbesondere zum Speichern von binär codierten Informationen.

Nach dem Stand der Technik ist es bekannt, Informationen, insbesondere binär codierte Informationen, auf ferromagnetischen Platten zu speichern. Solche ferromagnetischen Platten sind als Festplatten in Computern oder als Disketten weit verbreitet. Die Speicherkapazität pro Flächeneinheit solcher ferromagnetischer Platten ist durch die Kristallgröße der auf der Plattenoberfläche aufgebrachten magnetischen Kristalle begrenzt.

Andererseits ist es aus der US 5,507,834 bekannt, zum Nachweis einer Nukleotidsequenz eine weitere Nukleotidsequenz zu verwenden, die eine Sekundärstruktur aufweist. Bei der Sekundärstruktur handelt es sich um eine sogenannte Haarnadelschleife. An den gegenüberliegenden Schleifenabschnitten der Haarnadelschleife sind ein erstes und ein zweites fluorophores Molekül vorgesehen. Die fluorophoren Moleküle sind hier so ausgebildet, daß durch einen strahlungslosen oder durch einen direkten Energieübergang die Fluoreszenz gelöscht wird. Diese Effekte sind unter den Begriffen Förster-Effekt oder Fluoreszenz-Energie-Transfer (Science 279 (5354) 1998, 1228-1229) bekannt. Wenn die Haarnadelschleife geöffnet wird, geht die die Fluoreszenz löschende räumliche Beziehung zwischen dem ersten und dem zweiten fluorophoren Molekül verloren. Damit ist eine Fluoreszenz beobachtbar.

Aufgabe der vorliegenden Erfindung ist es, eine Vorrichtung und ein Verfahren zum Speichern von Informationen anzugeben, bei denen die Informationsdichte pro Flächeneinheit erhöht ist.

Diese Aufgabe wird durch die Merkmale der Ansprüche 1 und 10 gelöst. Zweckmäßige Weiterbildungen ergeben sich aus den Merkmalen der Ansprüche 2 bis 9 und 11 bis 19.

Nach Maßgabe der Erfindung ist eine Vorrichtung zum Speichern von Informationen vorgesehen, mit einem zur Ausbildung einer spezifischen Sekundärstruktur geeigneten Polymer, wobei das Polymer im Bereich der Sekundärstruktur ein erstes und ein damit in Wechselwirkung stehendes zweites fluorophores Molekül oder einen Quencher aufweist, wobei ein erstes Ende des Polymers an eine feste Phase gebunden und an dessen zweites Ende ein magnetisches oder ladungstragendes Mittel gebunden ist, und wobei bei Einwirkung eines äußeren magnetischen oder elektrischen Felds die Sekundärstruktur aufheb- oder so änderbar ist, daß die Wechselwirkung eliminierbar und damit eine Fluoreszenzreaktion erzeug- oder löschbar ist. - Die Informationsdichte pro Flächeneinheit einer solchen Vorrichtung ist drastisch erhöht.

Als Säuresequenz können geeignete Desoxiribonukleinsäure (=DNA), Peptidnukleinsäure (=PNA), Phosphothionatnukleinsäure (=PTO) oder Chimäre daraus, vorzugsweise aus DNA und PNA, verwendet werden. Es können anstatt einer Nukleotidsequenz auch andere Polymere, z. B. Proteine oder Peptide eingesetzt werden, die zur Ausbildung von spezifischen Sekundärstrukturen geeignet sind. Durch das Vorsehen von Sekundärstrukturen entlang der Sequenz lassen sich n+1 Zustände realisieren, deren Emissionsmaxima durch entsprechende Wahl der Donor/Akzep tor- bzw. Donor/Quencher-Paare verschieden sein können. Über die Wellenlänge und Intensität des Fluoreszenzsignals kann ermittelt werden, welcher der verschiedenen Zustände vorliegt.

Nach einer weiteren Lösung ist ein Verfahren von Speichern von Informationen mit einem zur Ausbildung einer spezifischen Sekundärstruktur geeigneten Polymer vorgesehen, wobei das Polymer ein erstes und ein damit in Wechselwirkung stehendes zweites fluorophores Molekül aufweist, wobei ein erstes Ende des Polymers an eine feste Phase gebunden und dessen zweites Ende ein magnetisches oder ladungstragendes Mittel aufweist, und wobei bei Einwirkung eines äußeren magnetischen oder elektrischen Felds die Sekundärstruktur aufgehoben oder so geändert wird, daß die Wechselwirkung eliminiert und damit eine Fluoreszenzreaktion erzeugt oder gelöscht wird. - Das Verfahren ermöglicht eine wesentliche Erhöhung der Informationsdichte pro Flächeneinheit.

Nachfolgend werden Ausführungsbeispiele der Erfindung anhand der Zeichnung näher erläutert. Hierin zeigen
Fig. 1 eine schematische Ansicht einer ersten Vorrichtung in einem ersten Zustand,
Fig. 2 die Vorrichtung nach Fig. 1 in einem zweiten Zustand,
Fig. 3 eine schematische Ansicht einer zweiten Vorrichtung in einem ersten Zustand,
Fig. 4 die Vorrichtung nach Fig. 3 in einem zweiten Zustand,
Fig. 5 eine schematische Ansicht einer dritten Vorrichtung in einem ersten Zustand,
Fig. 6 die Vorrichtung nach Fig. 5 in einem zweiten Zustand,
Fig. 7 eine schematische Ansicht einer vierten Vorrichtung in einem ersten Zustand,
Fig. 8 die Vorrichtung nach Fig. 7 in einem zweiten Zustand,
Fig. 9 eine schematische Ansicht einer fünften Vorrichtung in einem ersten Zustand,
Fig. 10 die Vorrichtung nach Fig. 9 in einem zweiten Zustand,
Fig. 11 eine schematische Ansicht einer sechsten Vorrichtung in einem ersten Zustand und
Fig. 12 die Vorrichtung nach Fig. 11 in einem zweiten Zustand,

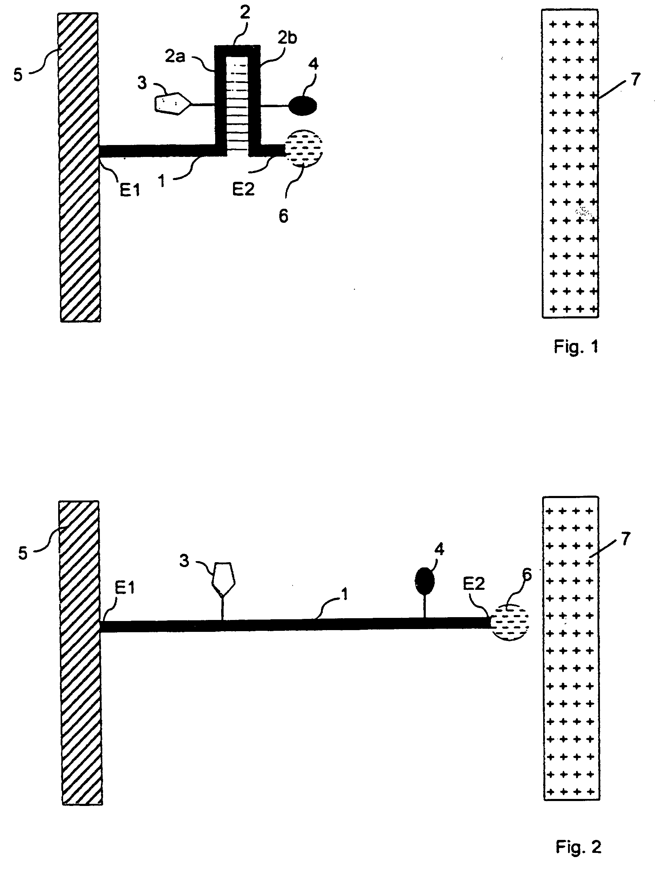

Fig. 1 zeigt schematisch eine erste Vorrichtung. Eine Sequenz 1 weist eine Haarnadelschleife 2 mit einem ersten 2a und einem korrespondierenden zweiten gegenüberliegenden Schleifenabschnitt 2b auf. Am ersten Schleifenabschnitt 2a ist eine erste fluorophore Gruppe 3 und am zweiten Schleifenabschnitt 2b ein Quencher 4 gebunden. Zwischen der ersten fluorophoren Gruppe 3 und dem Quencher 4 besteht eine Wechselwirkung, welche Fluoreszenz löscht oder die Wellenlänge des emittierten Lichts spezifisch verändert.

Ein erstes Ende E1 der Sequenz 1 ist an eine feste Phase 5, z.B. ein Polycarbonat, gebunden. An ein zweites Ende E2 der Sequenz 1 ist ein magnetisches Mittel 6 gekoppelt. Dabei kann es sich um eine paramagnetische Gruppe oder einen paramagnetischen Partikel handeln. Solche paramagnetische Partikel werden z. B. von den Firmen MILTENY oder DYNATECH angeboten. Sie können mittels einer Biotin/(Strept-)Avidin-, Amid-, Ester- oder Disulfid-Bindung an die Sequenz 1 gekoppelt werden. Gegenüberliegend der festen Phase 5 ist ein Magnet 7 angeordnet. Statt dem magnetischen Mittel kann auch ein elektrischer Ladungsträger und statt dem Magneten ein Mittel zur Erzeugung eines magnetischen Felds vorgesehen sein.

Statt des Quenchers 4 kann auch eine zweite fluorophore Gruppe vorgesehen sein. Dabei kann es sich um eine Donorgruppe handeln. In diesem Fall besteht die erste flourophore Gruppe aus einer Akzeptorgruppe. In der nachfolgenden Tabelle sind geeignete Donor-/Akzeptorverbindungen wiedergegeben:

| Donor | Akzeptor |
|---|---|
| Fluorescein | Fluorescein |
| 6-Carboxy-Fluorescein | 6-Carboxymethyl-Rhodamin |
| Fluorescein | Tetramethylrhodamin |
| IAEDANS (= 5-((((2-iodoacyl) amino)ethyl)amino)naphthalene-1-sulonsäure) | Fluorescein |
| EDANS (= 5-((2-aminomethyl) amino)naphthalen-1-sulfonsäure | DABCYL (4-dimethylaminoazobenzen-4'-sulfoylchlorid), |
| BODIPY FL | BODIPY FL |

In Fig. 2 befindet sich das magnetische Mittel 6 in der Nähe des Magneten 7. Die Haarnadelschleife 2 ist aufgehoben. Die erste fluorophore Gruppe 3 und der Quencher 4 sind soweit voneinander entfernt, daß keine Wechselwirkung mehr zwischen ihnen besteht.

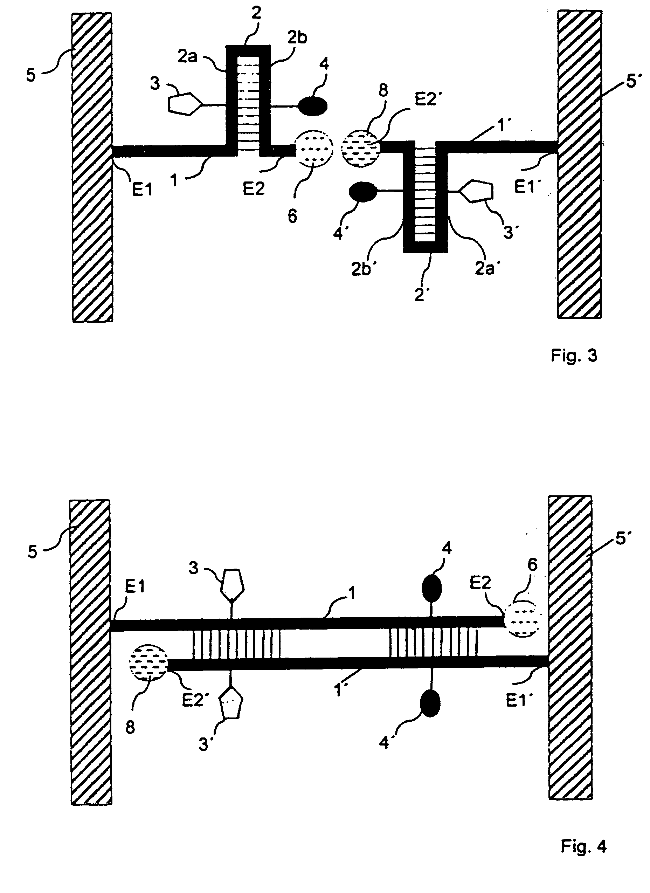

Fig. 3 zeigt eine schematische Ansicht einer zweiten Vorrichtung. Dabei befindet sich gegenüber einer Vorrichtung gemäß den Fig. 1 und 2 eine ähnliche weitere Vorrichtung. Diese unterscheidet sich dadurch, daß hier am zweiten Ende E2' der Nukleinsäuresequenz 1' ein zum magnetischen Mittel 6 entgegengesetzt polarisiertes weiteres magnetisches Mittel 8 gebunden ist.

Fig. 4 zeigt die Vorrichtung nach Fig. 3 in einem zweiten Zustand. Dabei befinden sich die Nukleinsäuresequenzen 1, 1' in einem langgestreckten Zustand, der durch die Ausbildung von Bindungen zwischen DNA-Molekülen der Nukleinsäuresequenzen 1 und 1' stabiliert wird.

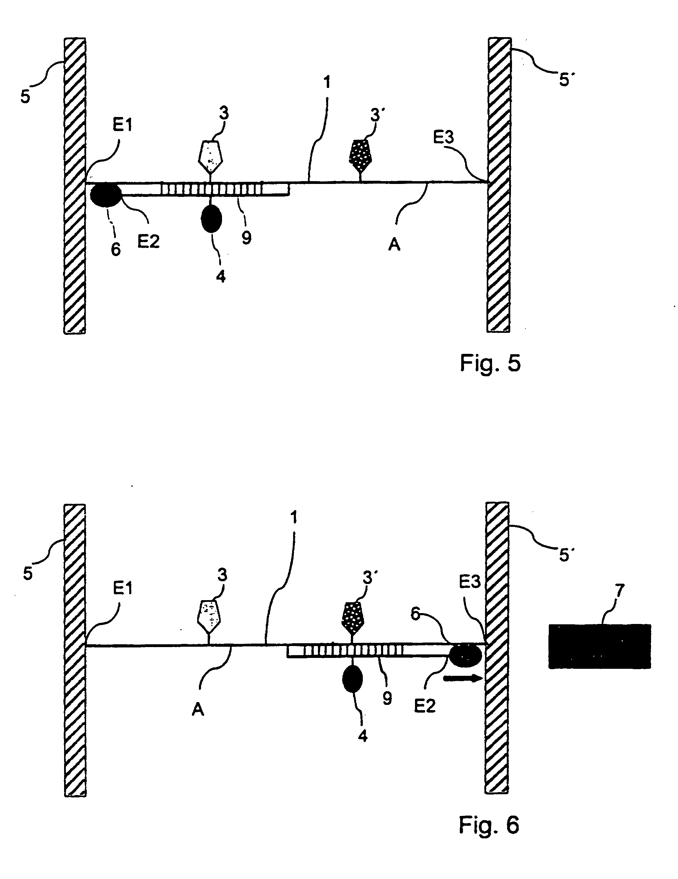

Die Fig. 5 und 6 zeigen eine dritte Vorrichtung in einem ersten bzw. zweiten Zustand. Dabei erstreckt sich ein Abschnitt A der Nukleinsäuresequenz 1 von einer festen Phase 5 zu einer gegenüberliegend in einem vorgegebenen Abstand angeordneten weiteren festen Phase 5'. Das erste Ende E1 der Nukleinsäuresequenz 1 ist an die feste Phase 5 und ein drittes Ende E3 an die weitere feste Phase 5' gebunden. Das zweite Ende E2 befindet sich an einem abzweigenden Ast 9. An das zweite Ende E2 ist das magnetische Mittel 6 gekoppelt. Der Ast 9 bildet mit dem Abschnitt A der Nukleinsäuresequenz 1 eine erste Sekundärstruktur, die in Fig. 5 gezeigt ist. Dabei befindet sich das magnetische Mittel 6 in der Nähe der festen Phase 5. Der Quencher 4 ist an den Ast 9 gebunden. Er befindet sich in der ersten Sekundärstruktur gegenüberliegend einer ersten am Abschnitt A gebundenen fluorophoren Gruppe 3. Fig. 6 zeigt die dritte Vorrichtung in einem zweiten Zustand, bei dem eine zweite Sekundärstruktur gebildet ist. Dabei befinden sich das magnetische Mittel 6 und ein Magnet 7 in der Nähe der weiteren festen Phase 5'. Der Quencher 4 befindet sich in der zweiten Sekundärstruktur gegenüberliegend einer an den Abschnitt A gebundenen weiteren fluorophoren Gruppe 3'.

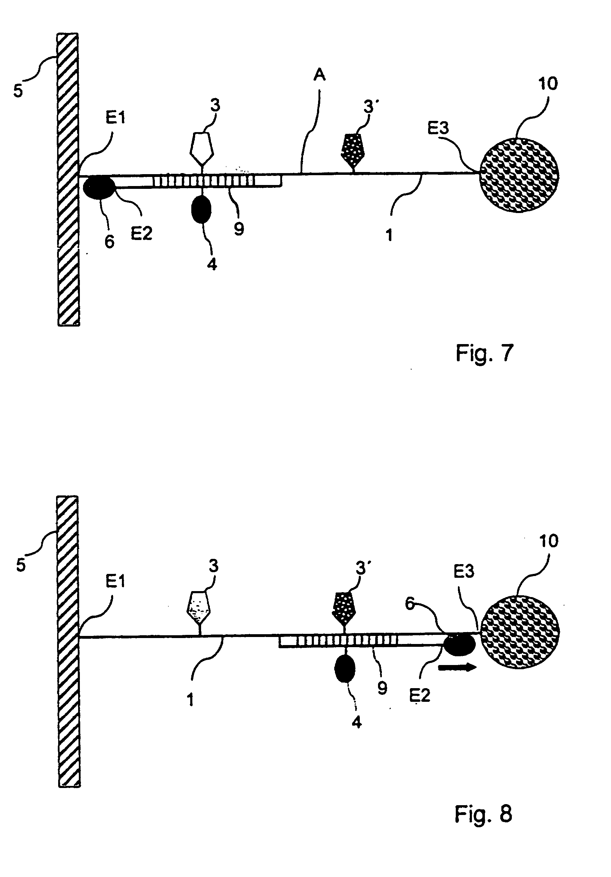

Die in den Fig. 7 und 8 gezeigte vierte Vorrichtung entspricht im wesentlichen der dritten Vorrichtung. Am dritten Ende E3 ist statt der weiteren festen Phase 5' ein Gruppe 10 gebunden, deren spezifisches Gewicht höher als das des umgebenden Milieus ist.

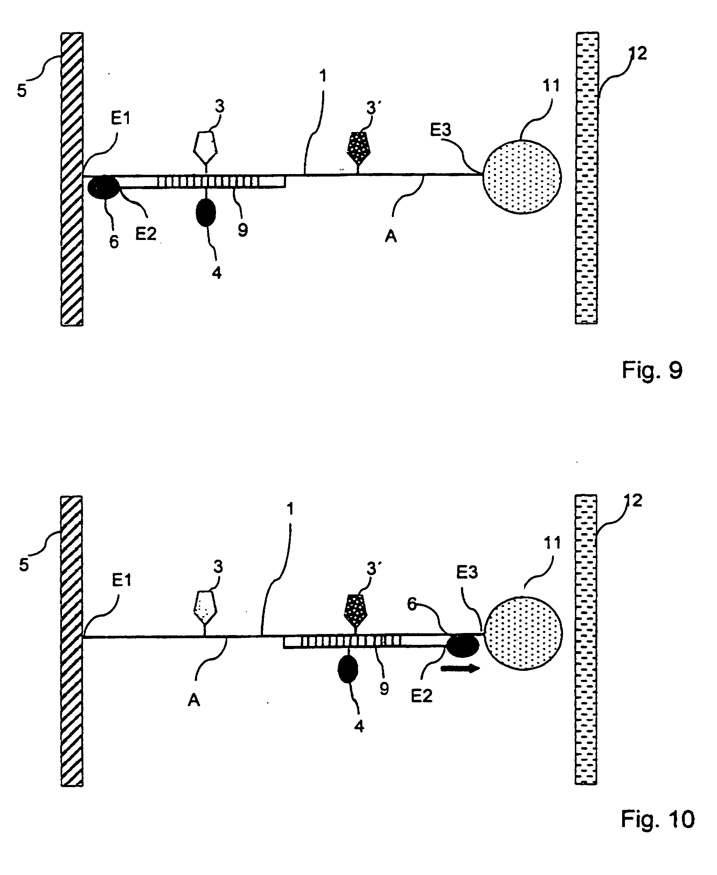

Die in den Fig. 9 und 10 gezeigte fünfte Vorrichtung entspricht im wesentlichen der in den Fig. 7 und 8 gezeigten vierten Vorrichtung. Dabei ist anstelle der Gruppe 10 ein elektrisch geladener Partikel 11 an das dritte Ende E3 gebunden.

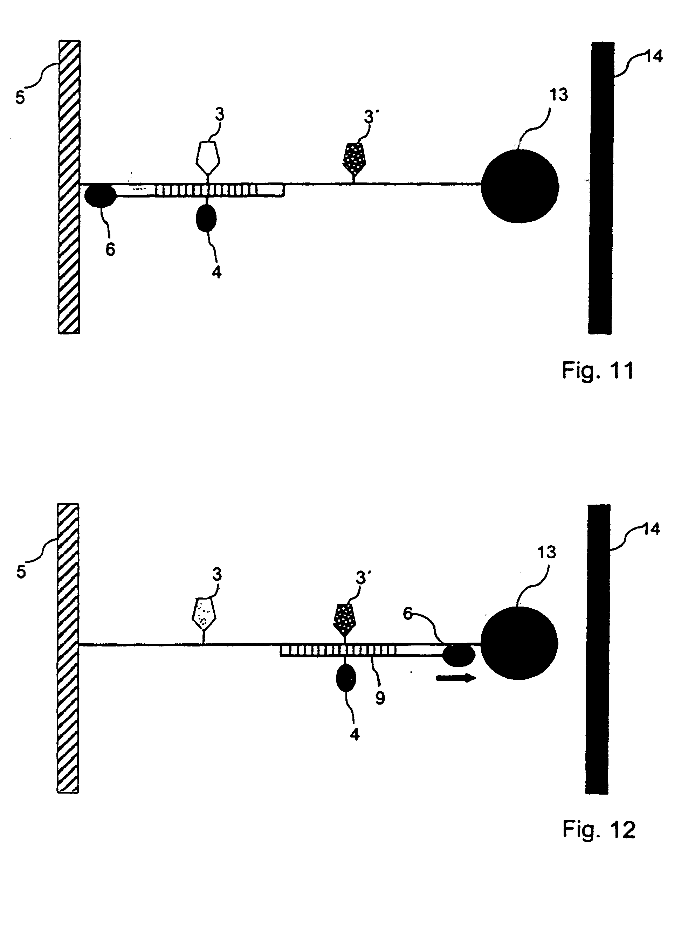

Die in den Fig. 11 und 12 gezeigte sechste Vorrichtung entspricht im wesentlichen der in den Fig. 9 und 10 gezeigten fünften Vorrichtung. Dabei ist anstelle des ladungstragenden Partikels eine magnetisierbare Gruppe 13 vorgesehen.

### Funktion der Vorrichtungen ist folgende:

Im in den Fig. 1 und 3 gezeigten Grundzustand weist die Nukleinsäuresequenz 1, 1' jeweils eine Haarnadelschleife 2, 2' auf. Die erste fluorophore Gruppe 3, 3' steht in Wechselwirkung mit dem gegenüberliegenden Quencher 4, 4'. Bei Anregung der ersten fluorophoren Gruppe 3, 3' wird durch Energieübergang auf den Quencher 4, 4' jegliche Fluoreszenz gelöscht. Bei Anlegen eines äußeren Magnetfelds, z.B. durch Aktivieren des Magneten 7, wird das magnetische Mittel 6, 8 vom ersten Ende E1, E1' wegbewegt. Die Haarnadelschleife 2, 2' wird aufgelöst. Die Sequenz 1 bildet nun ein langgestrecktes Molekül. Die Wechselwirkung zwischen der vormals gegenüberliegenden erste fluorophore Gruppe 3, 3' und dem Quencher 4, 4' fällt weg. Bei Anregung der ersten fluorophoren Gruppe 3, 3' ist nun eine Fluoreszenz beobachtbar. Die Fluoreszenz dient zum Nachweis des zweiten Zustands der Sequenz 1, 1'.

Zur Stabilisierung des zweiten Zustands können, wie in den Fig. 3 und 4 gezeigt ist, zwei Sequenzen 1, 1' einander gegenüberliegend angeordnet sein. Bei Anlegen eines äußeren Magnetfelds wird das magnetische Mittel 6 vom ersten Ende E1 wegbewegt. Das weitere magnetische Mittel 8 an der gegenüberliegenden Sequenz 1' wird infolge seiner entgegengesetzten Polarisierung vom weiteren ersten Ende E1' wegbewegt. Die Haarnadelschleifen 2, 2' werden aufgehoben. Die beiden Sequenzen 1, 1' stabilisieren sich gegenseitig durch die Ausbildung von Bindungen komplementärer Molekülbereiche.

Die Detektion der Zustände kann auch kapazitiv erfolgen. In diesem Fall werden anstelle des magnetischen Mittels 6 und des weiteren magnetischen Mittels 8 ladungstragende Gruppen verwendet.

In den Fig. 5 bis 12 wird jeweils durch den Ast 9 eine erste Sekundärstruktur gebildet, bei welcher der Quencher 4 entfernt von der weiteren ersten fluorophoren Gruppe 3' sich befindet. Bei Anregung der weiteren ersten fluorophoren Gruppe 3' ist eine Fluoreszenz beobachtbar. Unter dem Einfluß eines äußeren magnetischen oder elektrischen Felds wird das magnetische Mittel 6 von der festen Phase 5 wegbewegt; die erste Sekundärstruktur wird aufgehoben. Es bildet sich eine zweite Sekundärstruktur aus, bei der der Quencher 4 in Wechselwirkung mit der weiteren ersten fluorophoren Gruppe 3' tritt, so daß die dort beobachtbare Fluoreszenz gelöscht wird. Stattdessen tritt bei entsprechender Anregung nun eine Fluoreszenz an der ersten fluorophoren Gruppe 3 auf, die sich hinsichtlich ihrer Intensität oder Wellenlänge von der der weiteren ersten fluorophoren Gruppe 3' unterscheiden kann.

Zur Überführung des Asts 9 von der ersten in die zweite Sekundärstruktur können neben einem äußeren magnetischen oder elektrischen Feld gemäß Fig. 7 und 8 auch Zentrifugalkräfte dienen.

### Bezugszeichenliste

- 1, 1': Sequenz
- 2, 2': Haarnadelschleife
- 2a, 2a: erster Schleifenabschnitt
- 2b, 2b: zweiter Schleifenabschnitt
- 3, 3': erste fluorophore Gruppe
- 4, 4': Quencher
- 5, 5': feste Phase
- 6: magnetisches Mittel
- 7: Magnet
- 8: weiteres magnetisches Mittel
- 9: Ast
- 10: Gruppe
- 11: ladungstragender Partikel
- 12: polarisierbare Oberfläche
- 13: magnetisierbare Gruppe
- 14: magnetisierbare Oberfläche

- E1, E1': erstes Ende
- E2, E2': zweites Ende
- E3: drittes Ende
- A: Abschnitt

## Patentansprüche

1. Vorrichtung zum Speichern von Informationen mit einem zur Ausbildung einer spezifischen Sekundärstruktur geeigneten Polymer (1, 1'), wobei das Polymer (1, 1') im Bereich der Sekundärstruktur (2, 2') ein erstes (3, 3') und ein damit in Wechselwirkung stehendes zweites fluorophores Molekül oder Quencher (4, 4') aufweist, wobei ein erstes Ende (E1, E1') des Polymers (1, 1') an eine feste Phase (5, 5') gebunden und an dessen zweites Ende (E2, E2') ein magnetisches oder ladungstragendes Mittel (6, 8) gebunden ist, und wobei bei Einwirkung eines äußeren magnetischen oder elektrischen Felds die Sekundärstruktur (2, 2') aufheb- oder so änderbar ist, daß die besagte Wechselwirkung eliminierbar und damit eine Fluoreszenzreaktion erzeug- oder löschbar ist.

2. Vorrichtung nach Anspruch 1, wobei die Sekundärstruktur (2, 2') eine Haarnadelschleife, eine Helix, eine Triplex-Struktur, eine Faltblattstruktur oder ein Ring ist.

3. Vorrichtung nach Anspruch 2, wobei das erste fluorophore Molekül (3, 3') an einem ersten Schleifenabschnitt (2a, 2a') und das zweite fluorophore Molekül (4, 4') gegenüberliegend an einem zweiten Schleifenabschnitt (2b, 2b') der Haarnadelschleife (2, 2') in einem eine Wechselwirkung ermöglichenden Abstand gebunden sind.

4. Vorrichtung nach einem der vorhergehenden Ansprüche, wobei die feste Phase (5, 5') ein Kunststoff ist.

5. Vorrichtung nach Anspruch 4, wobei der Kunststoff ein Polycarbonat, Trimetylthiophen, Thiophen, Triaminobenzol und/oder ein Polycarben enthält.

6. Vorrichtung nach einem der vorhergehenden Ansprüche, wobei das erste fluorophore Molekül (3, 3') eine Akzeptorgruppe und das zweite fluorophore Molekül (4, 4') eine Donorgruppe ist.

7. Vorrichtung nach Anspruch 6, wobei die Akzeptorgruppe (3, 3') Tetramethylrhodamin, 6-Carboxy-Tetramethyl-Rhodamin, 4 Dimethylaminoazobenzen-4'sulfonylchlorid, BODYPY FL Dabayl oder Fluorescein ist.

8. Vorrichtung nach Anspruch 6 oder 7, wobei die Donorgruppe 6-Carboxy-Fluorescein, 5-((((2-iodoacyl)amino)ethyl)amino)naphthalen-1-sulfonsäure, 5-((2-aminomethyl)amino)naphthalen-1-sulfonsäure, BODYPY FL oder Fluorescein ist.

9. Vorrichtung nach einem der vorhergehenden Ansprüche, wobei das Polymer eine Nukleotidsequenz (1, 1'), DNA, Peptidumkleinsäure, Phosphathioratumkleinsäure, Peptid oder ein daraus gebildetes Chimär ist.

10. Verfahren zum Speichern von Informationen mit einem zur Ausbildung einer spezifischen Sekundärstruktur geeigneten Polymer (1, 1'), wobei das Polymer (1, 1') im Bereich der Sekundärstruktur (2, 2') ein erstes (3, 3') und ein damit in Wechselwirkung stehendes zweites fluorophores Molekül (4, 4') aufweist, wobei ein erstes Ende (E1, E1') des Polymers (1, 1') an eine feste Phase (5, 5') gebunden und dessen zweites Ende (E2, E2') ein magnetisches oder ladungstragendes Mittel (6, 8) aufweist, und wobei bei Einwirkung eines äußeren magnetischen oder elektrischen Felds die Sekundärstruktur (2, 2') aufgehoben oder so geändert wird, daß die besagte Wechselwirkung eliminiert und damit eine Fluoreszenzreaktion erzeugt oder gelöscht wird.

11. Verfahren nach Anspruch 10, wobei die Sekundärstruktur (2, 2') eine Haarnadelschleife, eine Helix, eine Triplex-Struktur, eine Faltblattstruktur oder ein Ring ist.

12. Verfahren nach Anspruch 11, wobei das erste fluorophore Molekül (3, 3') an einem ersten Schleifenabschnitt (2a, 2a') und das zweite fluorophore Molekül (4, 4') gegenüberliegend an einem zweiten Schleifenabschnitt (2b, 2b') der Haarnadelschleife (2, 2') in einem eine Wechselwirkung ermöglichenden Abstand gebunden sind.

13. Verfahren nach einem der Ansprüche 10 bis 12, wobei die feste Phase (5, 5') ein Kunststoff ist.

14. Verfahren nach Anspruch 13, wobei der Kunststoff ein Polycarbonat, Trimetylthiophen, Thiophen, Triaminobenzol und/oder ein Polycarben enthält.

15. Verfahren nach einem der Ansprüche 10 bis 14, wobei das erste fluorophore Molekül (3, 3') eine Akzeptorgruppe und das zweite fluorophore Molekül (4, 4') eine Donorgruppe ist.

16. Verfahren nach Anspruch 15, wobei die Akzeptorgruppe (3, 3') Tetramethylrhodamin, 6-Carboxy-Tetramethyl-Rhodamin, 4 Dimethylaminoazobenzen-4'sulfonylchlorid, BODYPY FL Dabayl oder Fluorescein ist.

17. Verfahren nach Anspruch 15 oder 16, wobei die Donorgruppe 6-Carboxy-Fluorescein, 5-((((2-iodoacyl)amino)ethyl)amino)naphthalen-1-sulfonsäure, 5-((2-aminomethyl)amino)naphthalen-1-sulfonsäure, BODYPY FL oder Fluorescein ist.

18. Verfahren nach einem der Ansprüche 10 bis 17 wobei das Polymer eine Nukleotidsequenz (1, 1'), DNA, Peptidumkleinsäure, Phosphotionatumkleinsäure, Peptid oder ein daraus gebildetes Chimär ist.

19. Verfahren nach einem der Ansprüche 10 bis 18, wobei die Fluoreszenz mittels eines mit einer Datenverarbeitungseinrichtung verbundenen Fluorometers erfaßt wird.

## Claims

1. Device for storing information with a polymer (1, 1') suitable to form a specific secondary structure, wherein the polymer (1, 1') has a first (3, 3') and a therewith interacting second fluorophore molecule or quencher (4, 4') in the area of the secondary structure (2, 2'), wherein a first end (E1, E1') of the polymer (1, 1') is bound to a fixed phase (5, 5') and to whose second end (E2, E2') a magnetic or charge-carrying agent (6, 8) is bound, and wherein the secondary structure (2, 2') can be canceled or changed during the influence of an exterior magnetic or electrical field, so that said interaction can be eliminated and thus a fluorescence reaction can be generated or quenched.

2. Device as defined in claim 1, wherein the secondary structure (2, 2') is a hair-pin loop, a helix, a triplex structure, a sheet structure or a ring.

3. Device as defined in claim 2, wherein the first fluorophore molecule (3, 3') is bound to a first loop section (2a, 2a') and the second fluorophore molecule (4, 4') is bound vis-à-vis to a second loop section (2b, 2b') of the hair-pin loop (2, 2') at an interval which permits interaction.

4. Device as defined in one of the preceding claims, wherein the fixed phase (5, 5') is a synthetic material.

5. Device as defined in claim 4, wherein the synthetic material contains a polycarbonate, trimethylthiophene, thiophene, triaminobenzol, and/or a polycarben.

6. Device as defined in one of the preceding claims, wherein the first fluorophore molecule (3, 3') is an acceptor group and the second fluorophore molecule (4, 4') is a donor group.

7. Device as defined in claim 6, wherein the acceptor group (3, 3') is tetramethyl-rhodamine, 6-carboxy-tetramethylrhodamine, 4 dimethylaminoazo benzen-4'sulphonylchoride, BODYPY FL dabayl or fluorescein.

8. Device as defined in claim 6 or 7, wherein the donor group is 6-carboxy-fluorescein, 5-((((2-iodoacyl)amino)ethyl)-amino)naphthalen-sulfone acid, 5-((2-aminomethyl)-amino) natphthalen-1-sulfone acid, BODYPY FL or fluorescein.

9. Device as defined in one of the preceding claims, wherein the polymer is a nucleotide sequence (1, 1'), DNA, peptide nucleic acid, phosphothionate nucleic acid, peptide or a chimera made thereof.

10. Method for storing information with a polymer (1, 1') suitable to form a specific secondary structure, wherein the polymer (1, 1') has a first (3, 3') and a therewith interacting second fluorophore molecule (4, 4') in the area of the secondary structure (2, 2'), wherein a first end (E1, E1') of the polymer (1, 1') is bound to a fixed phase (5, 5') and whose second end (E2, E2') has a magnetic or charge-carrying agent (6, 8) bound, and wherein the secondary structure (2, 2') is canceled or changed through the influence of an exterior magnetic or electrical field, so that said interaction is eliminated and thus a fluorescence reaction is generated or quenched.

11. Method as defined in claim 10, wherein the secondary structure (2, 2') is a hairpin-loop, a helix, a triplex structure, a folding leaf structure or a ring.

12. Method as defined in claim 11, wherein the first fluorophore molecule (3, 3') is bound to a first loop section (2a, 2a') and the second fluorophore molecule (4, 4') is bound vis-à-vis to a second loop section (2b, 2b') of the hair-pin loop (2, 2') at an interval which permits interaction.

13. Method as defined in one of the claims 10 to 12, wherein the fixed phase (5, 5') is a synthetic material.

14. Method as defined in claim 13, wherein the synthetic material contains a polycarbonate, trimethylthiophene, thiophene, triaminobenzol, and/or a polycarben.

15. Method as defined in one of the claims 10 to 14, wherein the first fluorophore molecule (3, 3') is an acceptor group and the second fluorophore molecule (4, 4') is a donor group.

16. Method as defined in claim 15, wherein the acceptor group (3, 3') is tetramethyl-rhodamine, 6-carboxytetramethyl-rhodamine, 4 dimethylaminoazobenzen-4'sulphonylchoride, BODYPY FL dabayl or fluorescein.

17. Method as defined in claim 15 or 16, wherein the donor group is 6-carboxy-fluorescein, 5-((((2-iodoacyl)amino)ethyl)-amino)naphthalen-sulfone acid, 5-((2-aminomethyl)-amino) naphthalen-1-sulfone acid, BODYPY FL or fluorescein.

18. Method as defined in one of the claims 10 to 17, wherein the polymer is a nucleotide sequence (1, 1'), DNA, peptide nucleic acid, phosphothionate nucleic acid, peptide or a chimera made thereof.

19. Method as defined in one of the claims 10 to 18, wherein the fluorescence is acquired via a fluorometer connected to a data processing facility.

## Revendications

1. Dispositif de mémorisation d'informations avec un polymère (1, 1') approprié pour la formation d'une structure secondaire spécifique, le polymère (1, 1') présentant dans la zone de la structure secondaire (2, 2') une première (3, 3') et ainsi une deuxième molécule fluorophore en interaction ou un quencher (4, 4'), une première extrémité (E1, E1') du polymère (1, 1') étant liée à une phase fixe (5, 5') et un agent magnétique ou porteur de charge (6, 8) étant lié à sa deuxième extrémité (E2, E2'), et la structure secondaire (2, 2'), sous l'action d'un champ magnétique ou électrique externe, étant supprimable ou modifiable de façon que ladite interaction puisse être éliminée et qu'une réaction de fluorescence puisse ainsi être générée ou supprimée.

2. Dispositif selon la revendication 1, la structure secondaire (2, 2') étant une boucle en épingle à cheveux, une hélice, une structure triplex, une structure en dépliant ou un anneau.

3. Dispositif selon la revendication 2, la première molécule fluorophore (3, 3') étant liée à une première partie de boucle (2a, 2a') et, à l'opposé, la deuxième molécule fluorophore (4, 4') étant liée à une deuxième partie de boucle (2b, 2b') de la boucle en épingle à cheveux (2, 2') à un intervalle permettant une interaction.

4. Dispositif selon l'une des revendications précédentes, la phase fixe (5, 5') étant une matière plastique.

5. Dispositif selon la revendication 4, la matière plastique contenant un polycarbonate, triméthylthiophène, thiophène, triaminobenzol et/ou un polycarbène.

6. Dispositif selon l'une des revendications précédentes, la première molécule fluorophore (3, 3') étant un groupe accepteur et la deuxième molécule fluorophore (4, 4') un groupe donneur.

7. Dispositif selon la revendication 6, le groupe accepteur (3, 3') étant du tétraméthyle de rhodamine, 6-carboxytétraméthyle de rhodamine, 4 diméthylaminoazobenzène-4 chlorure de sulfonyle, BODYPY FL Dabayl ou fluorescéine.

8. Dispositif selon la revendication 6 ou 7, le groupe donneur étant 6-carboxy-fluorescéine, 5-((((2-iodoacyle)amino)éthyle)-amino)naphthalene-1-acide sulfonique, 5-((2-aminométhyle)-amino)naphthalene-1-acide sulfonique, BODYPY FL ou fluorescéine.

9. Dispositif selon l'une des revendications précédentes, le polymère étant une séquence de nucléotide (1,1'), ADN, acide nucléique peptidique, phosho thionate acide nucléique, peptide ou une chimère en résultant.

10. Procédé de mémorisation d'informations avec un polymère (1, 1') approprié pour la formation d'une structure secondaire spécifique, le polymère (1, 1') présentant dans la zone de la structure secondaire (2, 2') une première (3, 3') et ainsi une deuxième molécule fluorophore (4, 4') en interaction, une première extrémité (E1, E1') du polymère (1, 1') étant liée à une phase fixe (5, 5') et sa deuxième extrémité (E2, E2') présentant un agent magnétique ou porteur de charge (6, 8), et la structure secondaire (2, 2'), sous l'action d'un champ magnétique ou électrique externe, étant supprimable ou modifiable de façon que ladite interaction soit éliminée, générant ou supprimant ainsi une réaction de fluorescence.

11. Procédé selon la revendication 10, la structure secondaire (2, 2') étant une boucle en épingle à cheveux, une hélice, une structure triplex, une structure en dépliant ou un anneau.

12. Procédé selon la revendication 11, la première molécule fluorophore (3, 3') étant liée à une première partie de boucle (2a, 2a') et, à l'opposé, la deuxième molécule fluorophore (4, 4') étant liée à une deuxième partie de boucle (2b, 2b') de la boucle en épingle à cheveux (2, 2') à un intervalle permettant une interaction.

13. Procédé selon l'une des revendications 10 à 12, la phase fixe (5, 5') étant une matière plastique.

14. Procédé selon la revendication 13, la matière plastique contenant un polycarbonate, triméthylthiophène, thiophène, triaminobenzol, et/ou un polycarbène.

15. Procédé selon l'une des revendications 10 à 14, la première molécule fluorophore (3, 3') étant un groupe accepteur et la deuxième molécule fluorophore (4, 4') un groupe donneur.

16. Procédé selon la revendication 15, le groupe accepteur (3, 3') étant du tétraméthyle de rhodamine, 6-carboxytétraméthyle de rhodamine, 4 diméthylaminoazobenzène-4 chlorure de sulfonyle, BODYPY FL Dabayl ou fluorescéine.

17. Procédé selon la revendication 15 ou 16, le groupe donneur étant 6-carboxy-fluorescéine, 5-((((2-iodoacyle)amino)éthyle)-amino)naphthalene-1-acide sulfonique, 5-((2-aminométhyle)-amino)naphthalene-1-acide sulfonique, BODYPY FL ou fluorescéine.

18. Procédé selon l'une des revendications 10 à 17, le polymère étant une séquence nucléotide (1, 1'), ADN, acide nucléide peptidique, phospho thionate acide nucléique, peptide ou une chimère en résultant.

19. Procédé selon l'une des revendications 10 à 18, la fluorescence étant saisie au moyen d'un fluorimètre relié à un dispositif de traitement de données.
